# FASCICULE DE BREVET EUROPEEN

(11) **EP 2 596 484 B1**
(45) Date de publication et mention de la délivrance du brevet: **08.07.2015**
(21) Numéro de dépôt: 11754893.3
(22) Date de dépôt: 19.07.2011
(51) Int. Cl.: G08C 17/02

(54) **SYSTÈME DE GESTION D'ÉQUIPEMENT D'UN BLOC OPÉRATOIRE ET UTILISATION CORRESPONDANTE**
SYSTEM ZUR VERWALTUNG DER ANLAGE EINES BEDIENBLOCKS UND ENTSPRECHENDE VERWENDUNG
SYSTEM FOR MANAGING EQUIPMENT OF AN OPERATIONAL BLOCK AND CORRESPONDING USE

(30) Priorité: 20.07.2010 FR 1055908
(43) Date de publication de la demande: 29.05.2013
(73) Titulaire: Maquet SAS, 45160 Ardon (FR)
(72) Inventeur: GUILLEMINOT, Bertrand, F-41240 Villermain (FR)
(74) Mandataire: Prugneau, Philippe
(86) Numéro de dépôt international: PCT/FR2011/051729
(87) Numéro de publication internationale: WO 2012/017163

(56) Documents cités:
- EP-A1- 1 480 180
- US-A1- 2009 080 348

## Description

La présente invention concerne un système de gestion d'équipement d'un bloc opératoire, le bloc opératoire comprenant au moins deux salles d'opération dont chaque salle d'opération comprend au moins un équipement, le système de gestion comprenant au moins une télécommande adaptée pour piloter l'au moins un équipement dans chacune des salles.

Un tel système est décrit dans le document EP 1 480 180.

Elle s'applique notamment au pilotage de l'équipement dans un bloc opératoire en minimisant le déplacement nécessaire de l'équipe médicale.

De l'état de la technique, on connaît des télécommandes comprenant un émetteur pour commander un appareil comprenant un récepteur. Ces télécommandes utilisent des ondes infrarouges pour la communication entre l'émetteur et le récepteur.

L'utilisation d'ondes infrarouges pour la communication par des télécommandes présente plusieurs inconvénients. Premièrement, une télécommande infrarouge devient inopérante si le trajet de propagation des ondes infrarouges est occulté par un obstacle, tel qu'un membre de l'équipe médicale ou un autre équipement de la salle d'opération. Deuxièmement, les ondes infrarouges ne sont pas adaptées à la transmission de données volumineuses, telles que des images. Troisièmement, avec un émetteur d'ondes infrarouges de faible puissance, l'utilisateur doit orienter l'émetteur de la télécommande correctement vers le récepteur de l'équipement pour pouvoir commander l'appareil ce qui peut être difficile ou impossible.

Dans le cas d'un émetteur d'ondes infrarouges de forte puissance, le troisième problème ne se présente pas. Par contre, dans ce cas, l'utilisation de télécommandes utilisant un simple protocole de communication par ondes infrarouges dans un système de gestion d'équipement d'un bloc opératoire avec plusieurs salles d'opération crée des risques de sécurité. Un tel risque est par exemple qu'un utilisateur commande un équipement autre que celui qu'il envisageait de commander, notamment un équipement dans une salle d'opération autre que celle dans laquelle l'utilisateur est destiné à travailler à un instant donné.

De l'état de la technique, on connaît également des panneaux de commande fixés à demeure par exemple à un mur, dans une salle d'opération pour le pilotage de l'équipement dans un bloc opératoire à distance, les panneaux de commande utilisant des radiofréquences. Pour le pilotage, ce système demande donc un déplacement d'un membre de l'équipe médicale jusqu'au panneau de commande.

La présente invention a pour but de proposer un système de gestion d'équipement d'un bloc opératoire qui est ergonomique et minimise le déplacement nécessaire de l'équipe médicale et qui ne présente pas au moins un des inconvénients mentionnés ci-dessus.

A cet effet l'invention a pour objet un système de gestion d'équipement d'un bloc opératoire du type précité, caractérisé en ce que le système de gestion comprend pour chaque télécommande :
- des moyens d'association associant la ou chaque télécommande exclusivement à l'une des salles d'opération de sorte à autoriser l'au moins une télécommande à piloter l'au moins un équipement dans la salle associée et à inhiber que l'au moins une télécommande pilote chaque équipement dans chacune des autres salles d'opération, auxquelles l'au moins une télécommande n'est pas associée ; et
- des moyens de modification adaptés pour modifier les moyens d'association de telle sorte que les moyens d'association associent l'au moins une télécommande exclusivement à une autre des au moins deux salles d'opération.

Selon d'autres modes de réalisation, le système de gestion selon l'invention comporte l'une ou plusieurs des caractéristiques suivantes :
- des moyens de communication entre la ou chaque télécommande et le ou chaque équipement dans chaque salle d'opération adaptés pour communiquer des signaux de commande de la télécommande au ou à chaque équipement ;
- les moyens de communication comprennent un premier émetteur disposé dans chaque télécommande et dans lequel chaque salle d'opération comporte au moins un premier récepteur correspondant, le premier émetteur et le premier récepteur utilisant une liaison de communication par radiofréquence afin de piloter l'équipement de la salle associée ;
- les moyens de communication définissent une première classe de commandes destinées à un équipement et une seconde classe de commandes destinées à cet équipement ;
- des moyens d'inhibition adaptés pour inhiber le pilotage de l'équipement par la télécommande lorsque l'équipement appartient à une salle d'opération autre que la salle à laquelle la télécommande est associée, mais uniquement lorsque la commande appartient à la première classe de commandes, mais non pas lorsque la commande appartient à la seconde classe de commandes ;
- des moyens d'autorisation qui sont adaptés pour autoriser la télécommande à piloter l'équipement dans la salle d'opération à laquelle la télécommande est associée uniquement lorsque la télécommande se trouve à l'intérieur d'un périmètre défini, par exemple à l'intérieur de la salle associée, et pour interdire à la télécommande de piloter l'équipement dans la salle d'opération à laquelle elle est associée lorsque la télécommande se trouve en dehors du périmètre défini ;
les moyens d'autorisation comprennent au moins l'un des suivants :
un dispositif de localisation de la télécommande, le dispositif de localisation utilisant notamment un principe de triangulation ;
un second émetteur utilisant une liaison de communication par radiofréquence avec une portée plus courte que celle du premier émetteur ;
un second émetteur utilisant une liaison de communication par ondes infrarouges vérifiant que la télécommande pointe sur l'équipement à commander ou sur le premier récepteur correspondant ;
un second émetteur utilisant une liaison de communication par ondes ultrasoniques,
le premier émetteur et des moyens de réglage de la portée, ce premier émetteur étant un émetteur dont la portée est modifiable et les moyens de réglage de la portée réglant la portée de telle sorte qu'elle est plus courte lorsque la commande appartient à la première classe que lorsque la commande appartient à la seconde classe ;
   - le système de gestion comprend une station d'association et des moyens de liaison de la télécommande à la station d'association, les moyens de modification étant adaptés pour modifier l'association lorsque la télécommande est reliée à la station d'association par les moyens de liaison ;
   - la station d'association comprend une interface homme - machine adaptée pour laisser un utilisateur choisir l'une des salles d'opération à laquelle il souhaite associer la télécommande ;
   - une station de confirmation adaptée pour confirmer l'association entre la télécommande et la salle et pour inhiber que la télécommande commande l'équipement de la salle associée avant que l'association soit confirmée ;
   - la station d'association et/ou la station de confirmation sont adaptées pour communiquer avec la ou chaque télécommande par une liaison du type « Bluetooth®», en particulier « Bluetooth 2.0® » ou ultérieur, ou du type « ZigBee » ; et
   - un dispositif de supervision disposé dans chaque salle d'opération, chaque dispositif de supervision étant relié à au moins l'un équipement, et de préférence à au moins deux équipements, dans la salle d'opération, le dispositif de supervision comprenant le premier récepteur correspondant et le cas échéant des moyens d'association dans lesquels est stockée une information d'association identifiant la ou les télécommande(s) associée(s) à cette salle d'opération.

L'invention a en outre pour objet une utilisation d'un système de gestion tel que précité pour gérer l'équipement d'un bloc opératoire, le bloc opératoire comprenant au moins deux salles d'opération, chaque salle d'opération comprenant au moins un équipement, l'utilisation comprenant les étapes suivantes :
- au moyen des moyens d'association associer la ou chaque télécommande exclusivement à l'une des au moins deux salles d'opération ;
- au moyen de la télécommande piloter l'équipement dans la salle d'opération associée et éventuellement inhiber le pilotage par cette télécommande de l'équipement dans chacune des au moins deux salles auxquelles la télécommande n'est pas associée ; et
- modifier l'association de la ou de chaque télécommande à l'une salle d'opération des au moins deux salles au moyen des moyens de modification en associant la télécommande à une autre des au moins deux salles d'opération.

L'invention sera mieux comprise à la lecture de la description qui va suivre, donnée uniquement à titre d'exemple et faite en se référant aux dessins annexés, sur lesquels :
- la figure 1 est une vue schématique d'un système de gestion d'équipement d'un bloc opératoire selon l'invention, comprenant trois télécommandes, dont deux télécommandes sont autorisées au pilotage d'un équipement associé,
- la figure 2 est une vue schématique détaillée de l'une des télécommandes de la figure 1, et
- la figure 3 est une vue schématique du système de gestion d'équipement d'un bloc opératoire de la figure 1, les deux télécommandes étant inhibées du pilotage de l'équipement associé.

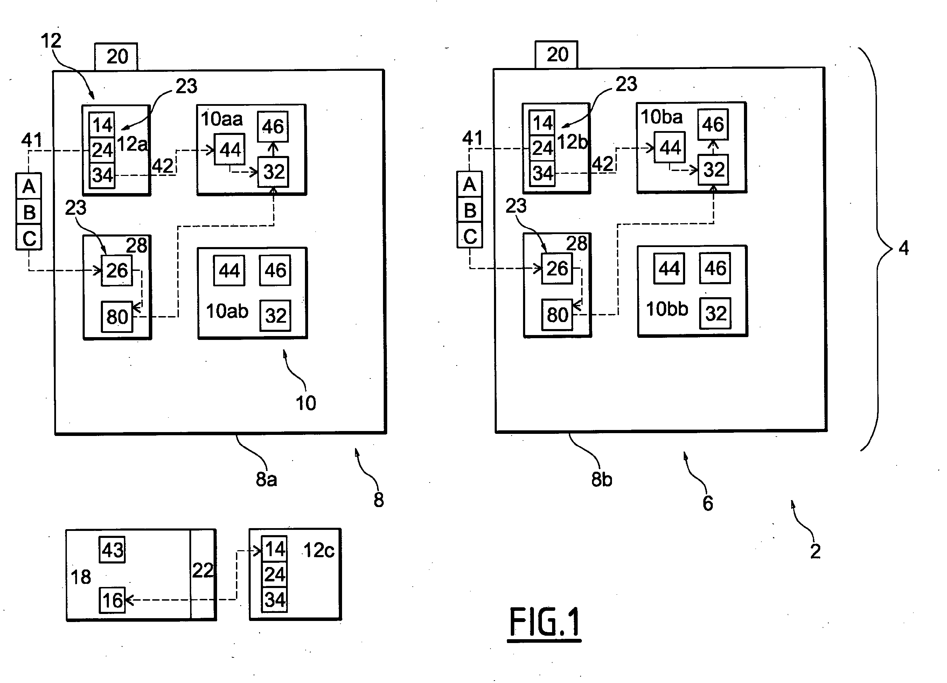

Sur la figure 1 est représenté un ensemble de gestion, désigné par la référence générale 2, l'ensemble de gestion 2 comprend un bloc opératoire 4 et un premier mode de réalisation d'un système de gestion d'équipement 6 du bloc opératoire 4. A titre d'exemple, le bloc opératoire 4 comprend deux salles d'opération 8, à savoir les salles d'opération 8a et 8b. Chaque salle d'opération 8 comprend deux équipements 10. La salle 8a comprend les équipements 10aa et 10ab. La salle 8b comprend les équipements 10ba et 10bb. Néanmoins, le bloc opératoire 4 peut comprendre un nombre quelconque de salles d'opération 8 qui peuvent comprendre chacun un nombre quelconque d'équipements 10. Chaque salle d'opération 8 est caractérisée par une adresse unique.

Sous « équipement » » 10 on entend tout appareil électronique et/ou mécatronique adapté pour effectuer une fonction suite à une commande d'un utilisateur, tel qu'un éclairage opératoire, un équipement audiovisuel (moniteur, caméra), un bras de support motorisé, un bras de chirurgie assisté par ordinateur, une table d'opération (notamment la hauteur ou la position de la table) ou des stores adaptés pour voiler des murs transparents de la salle d'opération.

Le système de gestion 6 comprend au moins une télécommande 12, ainsi que pour chaque télécommande 12 des moyens d'association 14 et des moyens de modification 16. Le système de gestion 6 comprend en outre au moins une station d'association 18, au moins une station de confirmation 20, des moyens de liaison 22, des moyens de communication 23 sous forme d'un premier émetteur 24 et d'un premier récepteur 26, pour chaque salle d'opération 8 au moins un dispositif de supervision 28, des moyens d'inhibition 30 et des moyens d'autorisation 32.

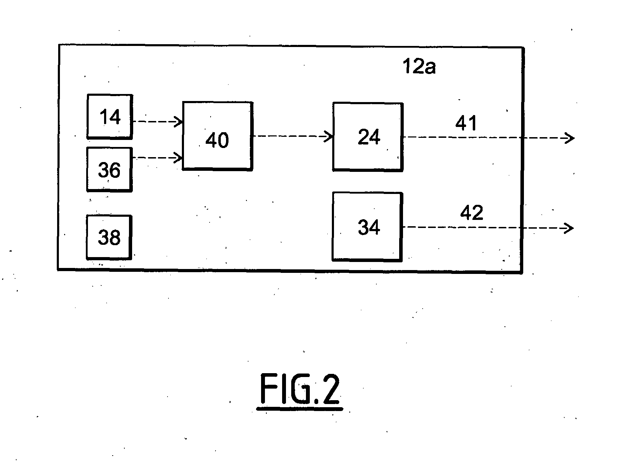

Dans l'exemple représenté sur la figure 1, le système de gestion 6 comprend trois télécommandes 12a, 12b, 12c. A titre d'exemple, la télécommande 12a est représentée en détail sur la figure 2. Chaque télécommande 12 comprend un premier émetteur 24 des moyens de communication 23, un second émetteur 34 et des moyens d'association 14. Chaque télécommande 12 comprend en outre un générateur de commandes 36, une batterie 38 rechargeable et un module de vérification 40 (figure 2). Le premier émetteur 24 est adapté pour émettre un signal 41 représentant une commande. Le signal de commande 41 comprend les informations suivantes (Figure 1) :
- A : une association entre la télécommande 12 et l'une des salles d'opération 8,
- B : l'équipement 10 pour lequel la commande est destinée, et
- C : la commande à exécuter.

Le second émetteur 34 est adapté pour émettre un signal de localisation 42. Le module de vérification 40 est adapté pour vérifier si une association de la télécommande 12 à une salle d'opération 8 a été confirmée au moyen de l'une des stations de confirmation 20. Le générateur de commandes 36 est adapté pour générer les signaux de commandes 41.

Chaque télécommande 12 est adaptée pour piloter un ou plusieurs des équipements 10 dans chacune des salles d'opération 8. De préférence, chaque télécommande 12 est adaptée pour piloter chaque équipement 10 dans chacune des salles d'opération 8. Chaque télécommande 12 est caractérisée par un identifiant unique.

Les moyens d'association 14 sont adaptés pour stocker des informations représentant une ou plusieurs association(s) exclusive(s) entre l'identifiant unique d'une télécommande 12 et l'adresse unique d'une des salles d'opération 8. Par cette information d'association, les moyens d'association 14 sont adaptés pour autoriser la télécommande 12 à piloter l'équipement 10 de la salle d'opération 8 associée et pour inhiber le pilotage par la télécommande 12 de l'équipement 10 de l'autre salle d'opération 8 non-associée.

Selon une première variante du premier mode de réalisation, les moyens d'association 14 comprennent une mémoire centrale à laquelle sont reliés tous les éléments du système de gestion 6 qui requièrent les informations d'association. Selon une seconde variante du premier mode de réalisation, les moyens d'association 14 sont intégrés dans tous les éléments du système de gestion 6 qui requièrent les informations d'association. Ces éléments sont les télécommandes 12, les moyens de modification 16, les stations d'association 18, les stations de confirmation 20, les moyens de communication 23, les dispositifs de supervision 28, les moyens d'inhibition 30 et les moyens d'autorisation 32.

Les moyens de modification 16 sont adaptés pour modifier les informations représentant la ou les association(s) stockées dans les moyens d'association 14. Les moyens de modification 16 comprennent par exemple un processeur et une mémoire comprenant un programme.

Chaque station d'association 18 est adaptée pour associer de manière modifiable chaque télécommande 12 exclusivement à l'une parmi les deux salles d'opération 8 et pour modifier une telle association. Les stations d'association 18 comprennent une interface homme - machine 43 adaptée pour laisser un utilisateur choisir une salle d'opération 8 à laquelle il souhaite associer la télécommande 12. A cet effet, les stations d'association 18 sont reliées aux moyens de modification 16 ou intègrent les moyens de modification 16. Les stations d'association 18 se présentent par exemple sous forme de pupitres. Les stations d'association 18 sont agencées à l'extérieur des salles d'opération 8. Les stations d'association 18 sont en outre adaptées pour mettre à jour les télécommandes 12 lors d'une mise à jour du système de gestion 6.

Le système de gestion 6 et/ou les stations d'association 18 sont adaptés pour modifier l'association de la télécommande en réponse au choix d'association par l'utilisateur.

Chaque station d'association 18 comprend également des moyens de recharge (non représentés) des batteries 38 rechargeables. Ces moyens de recharge sont par exemple des moyens de recharge par induction. Les moyens de recharge comprennent une première bobine d'induction connectée aux batteries 38 et une deuxième bobine de recharge disposée dans la station d'association 18. Ainsi, lorsque les télécommandes 12 sont reliées à l'une des stations d'association 18 en vue de modifier l'association, simultanément les batteries 38 sont rechargées.

En variante, les stations d'association 18 ne sont pas des stations physiques mais des zones géographiques prédéterminées à l'intérieur desquelles l'utilisateur associe sans contact physique entre la télécommande 12 et la station d'association 18 chaque télécommande 12 exclusivement à l'une parmi les deux salles d'opération 8. Dans ce cas, l'interface homme - machine 43 est intégrée dans chaque télécommande 12, et les moyens de recharge sont séparés des stations d'association 18.

Les stations de confirmation 20 sont adaptées pour confirmer l'association faite à l'une des stations d'association 18. Une télécommande 12 associée à une salle d'opération 8 n'est autorisée à piloter l'équipement 10 dans la salle d'opération 8 associée qu'après confirmation de cette association au moyen de l'une des stations de confirmation 20. Les stations de confirmation 20 sont agencées, soit ensemble avec les stations d'association 18, soit à l'entrée de chaque salle d'opération 8, soit entre ces deux endroits.

Les moyens de liaison 22 sont adaptés pour établir une connexion, au moins informatique, entre les télécommandes 12 et la station d'association 18, ainsi qu'entre les télécommandes 12 et la station de confirmation 20. Cette connexion relie la station d'association 18 et la station de confirmation 20 aux moyens d'association 14 intégrés dans les télécommandes 12. La connexion est adaptée à la transmission des signaux représentant l'association « salle d'opération 8 - télécommande 12 ». De préférence, la connexion est une connexion du type « Bluetooth® », en particulier « Bluetooth 2.0® » ou ultérieur. Les moyens de liaison 22 sont intégrés dans la station d'association 18 et dans la station de confirmation 20.

Le standard « Bluetooth® » répond à la norme IEEE802.15.1 ou est identique à celle-ci.

En variante, la connexion est une connexion selon le standard « ZigBee » ou répondant à la norme IEEE802.15.4.

Les moyens de communication 23 sont adaptés pour établir et assurer une communication, notamment un transfert de signaux de commande 41, entre la télécommande 12 et l'équipement 10 de la salle d'opération 8 associée en utilisant un protocole de communication par radiofréquence. Les moyens de communication 23 sont les premiers émetteurs 24 et les premiers récepteurs 26. Les premiers émetteurs 24 sont intégrés dans chaque télécommande 12. Les premiers récepteurs 26 sont intégrés dans chaque dispositif de supervision 28. Les moyens de communication 23 sont en outre adaptés pour définir des classes de commande. Les moyens de communication 23 sont aussi adaptés pour identifier la classe à laquelle appartient une commande C donnée. A titre d'exemple, on distingue, une première classe de commande et une seconde classe de commande. La première classe de commandes est constituée de commandes C dites « critiques ». Les commandes C critiques sont des commandes C dont l'exécution est potentiellement dangereuse pour le patient ou le personnel. La seconde classe de commande comprend toutes autres commandes C.

Chaque dispositif de supervision 28 est adapté pour recevoir les signaux de commande 41 provenant du premier émetteur 24 de chaque télécommande 12. Le dispositif de supervision 28 comprend le premier récepteur 26. Le dispositif de supervision 28 est relié à un ou plusieurs équipements 10 de la même salle 8 et adapté pour transmettre les signaux de commande 41 à l'équipement 10 correspondant.

En variante, le dispositif de supervision 28 est relié à tous les équipements 10 d'une salle d'opération 8 et adapté pour transmettre les signaux de commande 41 à tous ces équipements 10. Le dispositif de supervision 28 prend le rôle de récepteur de signaux de commande 41 dit « collectif » pour tous les équipements 10 reliés au dispositif de supervision 28. Dans chaque salle d'opération 8 est prévu un ou plusieurs dispositif(s) de supervision 28.

Les moyens d'inhibition 30 sont adaptés, lorsque la commande C appartient à la première classe de commandes, pour inhiber le pilotage de l'équipement 10 par la télécommande 12 lorsque l'équipement 10 appartient à une salle d'opération 8 autre que la salle d'opération 8 à laquelle la télécommande 12 est associée. Lorsque la commande C appartient à la deuxième classe de commandes, les moyens d'inhibition 30 ne sont pas adaptés pour inhiber le pilotage de l'équipement 10 par la télécommande 12 lorsque l'équipement 10 appartient à une salle d'opération 8 autre que la salle d'opération 8 à laquelle la télécommande 12 est associée. Les moyens d'inhibition 30 fonctionnent donc en fonction de la classe de la commande. Les moyens d'inhibition 30 sont intégrés dans le dispositif de supervision 28. Les moyens d'inhibition 30 sont reliés au premier récepteur 26.

Les moyens d'autorisation 32 sont adaptés pour autoriser la télécommande 12 à piloter le ou les équipement(s) 10 de la salle d'opération 8 associée lorsque la télécommande 12 se trouve dans un périmètre prédéfini et lorsque la commande C appartient à la première classe de commandes. Les moyens d'autorisation 32 sont également adaptés pour inhiber le pilotage par la télécommande 12 du ou des équipement(s) 10 de la salle d'opération 8 associée lorsque la télécommande 12 se trouve en dehors du périmètre prédéfini et lorsque la commande C appartient à la première classe de commandes. Les moyens d'autorisation 32 fonctionnent donc en fonction de la localisation de la télécommande 12 par rapport au périmètre prédéfini. A cet effet, les moyens d'autorisation 32 sont reliés aux moyens d'inhibition 30.

Les moyens d'autorisation 32 sont en outre adaptés pour localiser une télécommande 12 émettant un signal de localisation 42 et pour déterminer la position de cette télécommande 12 par rapport au périmètre prédéfini. Le périmètre prédéfini a sensiblement la forme d'une sphère avec un rayon de 5 mètres. Typiquement, le périmètre prédéfini englobe la salle d'opération 8 correspondante. Néanmoins, il est tout à fait possible de définir un périmètre plus petit que la salle d'opération 8 correspondante. A cet effet, les moyens d'autorisation 32 comprennent au moins l'un des suivants :
- Les moyens d'autorisation 32 comprennent un dispositif de localisation de la télécommande 12. Ce dispositif de localisation est adapté pour déterminer une position de la télécommande 12 par triangulation.
- Les moyens d'autorisation 32 comprennent un second récepteur 44 adapté pour recevoir les signaux de localisation 42 du second émetteur 34 de la télécommande 12, les seconds récepteurs 44 et émetteurs 34 utilisant une liaison de communication par radiofréquence avec une portée plus courte que celle du premier émetteur 24 et du premier récepteur 26. La portée de la liaison entre les seconds émetteurs 34 et récepteurs 44 détermine ledit périmètre.

En variante, les seconds récepteurs 44 et émetteurs 34 utilisent une liaison de communication par ondes infrarouges.

En variante encore, les seconds récepteurs 44 et émetteurs 34 utilisent une liaison de communication par ondes ultrasoniques.

Le second récepteur 44 des moyens d'autorisation 32 est intégré dans chaque équipement 10.

Chaque équipement 10 comprend en outre un convertisseur 46 adapté pour traduire une commande en une fonction correspondante de l'équipement 10.

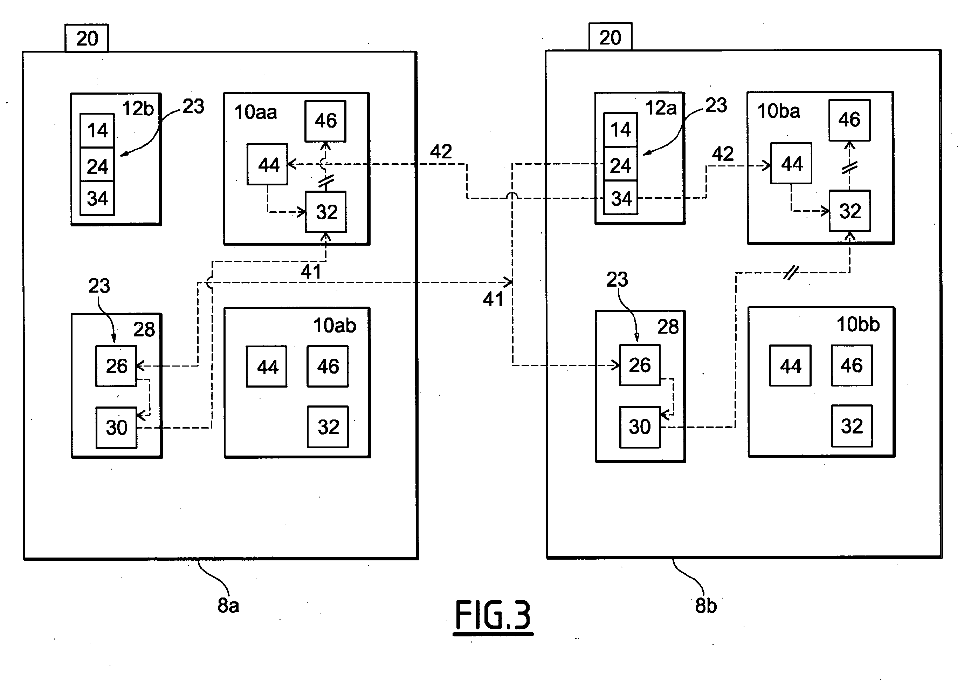

Dans ce qui suit, l'utilisation du système de gestion 6 sera décrite en référence aux figures 1 et 3.

Avant d'entrer dans l'une des deux salles d'opération 8 du bloc opératoire 4, l'utilisateur choisit l'une des télécommandes 12, par exemple la télécommande 12a. Au moyen des moyens de liaison 22, la télécommande 12a est reliée à l'une des stations d'association 18. L'interface 43 présente à l'utilisateur toutes les salles d'opération 8 auxquelles la télécommande 12a peut être associée. L'utilisateur choisit l'une des salles 8, par exemple la salle 8a, et effectue l'association entre l'adresse unique de la salle 8a et l'identifiant unique de la télécommande 12a. Ensuite, les moyens de modification 16 modifient cette information d'association pour la télécommande 12a et l'information d'association est stockée dans les moyens d'association 14. Cette association est valable jusqu'à ce qu'une nouvelle association soit faite.

A cette étape, la télécommande 12a est encore inhibée de piloter l'équipement 10aa et 10ab de la salle 8a par la station de confirmation 20. Pour des raisons de sécurité, le système de gestion 6 demande une confirmation de l'association entre la salle 8a et la télécommande 12a avant d'annuler l'inhibition. Au moyen des moyens de liaison 22, l'utilisateur relie la télécommande 12a à la station de confirmation 20 et confirme l'association la plus récente de la télécommande 12a stockée dans les moyens d'association 14. En conséquence, l'inhibition est supprimée par la station de confirmation 20.

Dans la salle associée 8a, l'utilisateur souhaite par exemple piloter l'équipement 10aa. Le premier émetteur 24 de la télécommande 12a émet un signal de commande 41 correspondant de radiofréquence. Le signal de commande 41 est transmis au premier récepteur 26 du dispositif de supervision 28 de la salle 8a. Les moyens de communication 23 identifient la classe de commandes à laquelle la commande C représentée par le signal de commande 41 appartient. Les moyens d'inhibition 30 autorisent la télécommande 12a à commander l'équipement 10aa lorsque la commande du signal 41 appartient à la première classe de commandes et lorsque la télécommande 12a émettant ce signal 41 est associée à la salle d'opération 8a dans laquelle se trouve l'équipement 10aa à commander (figure 1). Dans ce cas, le pilotage par cette télécommande 12a de tout autre équipement 10ab présent dans la salle d'opération 8a à laquelle la télécommande 12a est associée est également autorisé.

Supposons que l'utilisateur est fortuitement rentré dans la salle 8b à la quelle la télécommande 12a n'est pas associée. Au cas où la commande C appartient à la première classe de commandes, mais la télécommande 12a émettant le signal 41 n'est pas associée à la salle d'opération 8b dans laquelle se trouve l'équipement 10ba que l'utilisateur souhaite commander, les moyens d'inhibition 30 inhibent le pilotage par cette télécommande 12a de l'équipement 10ba présent dans la salle d'opération 8b dans laquelle la télécommande 12a se trouve (figure 3). Dans ce cas, le pilotage par télécommande 12a de tout autre équipement 10bb présent dans la salle d'opération 8b à laquelle la télécommande 12a n'est pas associée est également inhibé.

Au cas où la commande C appartient à la seconde classe de commandes, les moyens d'inhibition 30 toujours autorisent la télécommande 12a à commander l'équipement 10aa et tout autre équipement 10ab, 10ba, 10bb.

Les moyens d'autorisation 32 localisent la télécommande 12a et déterminent si la télécommande 12a se trouve à l'intérieur ou à l'extérieur du périmètre prédéfini, notamment à l'intérieur ou à l'extérieur de la salle d'opération 8a.

La localisation de la télécommande 12a est réalisée par l'un des principes suivants ou par une combinaison de plusieurs de ces principes.
- Le dispositif de localisation détermine la position de la télécommande 12a par rapport à des points de référence fixes dans la salle d'opération 8a par triangulation. Les moyens d'autorisation 32 déterminent si la position de la télécommande 12a est à l'intérieur ou à l'extérieur du périmètre prédéterminé.
- Le second émetteur 34 de la télécommande 12a émet un signal de localisation 42 de radiofréquence dont la portée est limitée par rapport au signal de commande 41 de radiofréquence. Si la distance entre le second émetteur 34 de la télécommande 12a et le second récepteur 44 des moyens d'autorisation 32 de l'équipement 10aa est plus courte que ou égale à la portée limitée du signal de localisation 42 de radiofréquence, le signal de localisation 42 de radiofréquence est transmis du second émetteur 34 au second récepteur 44. La télécommande 12a est donc considérée comme étant dans le périmètre prédéterminé. Par contre, si la distance entre le second émetteur 34 de la télécommande 12a et le second récepteur 44 des moyens d'autorisation 32 de l'équipement 10aa est plus longue que la portée limitée du signal de localisation 42 de radiofréquence, le signal de localisation 42 de radiofréquence n'est pas transmis du second émetteur 34 au second récepteur 44. La télécommande 12a est donc considérée comme n'étant pas dans le périmètre prédéterminé.
- Le second émetteur 34 de la télécommande 12a émet un signal de localisation 42 d'une longueur d'ondes des ondes infrarouges. La télécommande 12a est considérée comme étant dans le périmètre prédéterminé si les ondes infrarouges sont transmises du second émetteur 34 jusqu'au second récepteur 44 des moyens d'autorisation 32 de l'équipement 10aa. Les ondes infrarouges sont transmises du second émetteur 34 au second récepteur 44 uniquement si leur trajet n'est pas occulté par un objet.
- Le second émetteur 34 de la télécommande 12a émet un signal de localisation 42 à ultrasons dont la portée est limitée de sorte à couvrir un périmètre prédéterminé. La télécommande est considérée comme étant dans le périmètre prédéterminé si les ondes ultrasoniques sont transmises du second émetteur au second récepteur des moyens d'autorisation avec au moins une puissance déterminée.

Lorsque la commande C appartient à la première classe de commandes, les moyens d'inhibition 30 autorisent la télécommande 12a à commander l'équipement 10aa, et lorsque la télécommande 12a se trouve à l'intérieur du périmètre prédéterminé, notamment dans la salle 8a, les moyens d'autorisation 32 autorisent également la télécommande 12a à piloter au moins l'équipement 10aa (figure 1). Dans ce cas, de préférence, les moyens d'autorisation 32 autorisent la télécommande 12a à piloter tous les équipements 10aa, 10ab présents dans la salle 8a.

Lorsque la commande C appartient à la première classe de commandes, les moyens d'inhibition 30 autorisent la télécommande 12a à commander l'équipement 10, et lorsque la télécommande 12a se trouve à l'extérieur du périmètre prédéterminé, notamment dans la salle 8b, le pilotage de l'équipement 10aa par la télécommande 12a est inhibé au moyen des moyens d'autorisation 32 (figure 3).

Lorsque la commande appartient à la seconde classe de commandes, les moyens d'autorisation 32 autorisent la télécommande 12a à piloter l'équipement 10aa, indépendamment de la position de la télécommande 12a par rapport au périmètre prédéterminé.

C'est-à-dire, lorsque la commande C appartient à la première classe de commandes, la télécommande 12 doit a) être associée à la salle 8 dans laquelle se trouve l'équipement 10 à commander et b) se trouver à l'intérieur du périmètre prédéterminé afin d'autoriser la télécommande 12 à commander l'équipement 10 (figure 1). Si l'une des conditions a) ou b) ou les deux conditions ne sont pas satisfaites, la télécommande 12 n'est pas autorisée à commander cet équipement 10 (figure 3).

Lorsque la commande appartient à la seconde classe de commandes, la télécommande 12 est autorisée à commander l'équipement 10, indépendamment des conditions a) et b).

Dans l'exemple représenté sur la figure 1, le système de gestion 6 comprend trois télécommandes 12a, 12b, 12c et deux salles d'opération 8a, 8b, chaque salle 8 étant associée à une seule télécommande 12. Néanmoins, le système de gestion 6 selon l'invention est adapté pour l'association de la même salle 8 à un nombre quelconque de télécommandes 12. Ceci permet que plusieurs utilisateurs puissent piloter l'équipement 10 dans la même salle 8. Par contre, une télécommande 12 est associée soit à aucune salle 8, soit exclusivement à une seule salle 8.

On notera que chaque télécommande 12 est adaptée pour piloter chaque équipement 10 dans le bloc opératoire 4. Néanmoins, pour les commandes C appartenant à la première classe de commandes, les conditions a) et b) ci-dessus doivent être satisfaites afin d'autoriser les télécommandes 12 à piloter l'un des équipements 10. Par conséquent, pendant que la télécommande 12a est associée à la salle 8a, la télécommande 12a n'est jamais adaptée pour piloter ni tout l'ensemble de l'équipement 10 du bloc opératoire 4, ni deux équipements 10ab, 10bb appartenant à deux salles d'opération 8a, 8b différentes.

Selon un deuxième mode de réalisation, qui diffère du premier mode de réalisation uniquement par ce qui suit, le système de gestion 6 ne comprend pas de dispositif de supervision 28 ou seulement dans quelques-unes des salles d'opération 8. Pour les salles 8 dépourvues de dispositif de supervision 28, les premiers récepteurs 26, recevant les signaux de commande 41 et les moyens d'inhibition 30 sont intégrés dans chaque équipement 10 de ces salles 8. Dans ce cas, chaque équipement 10 comprend le premier récepteur 26 pour recevoir les signaux de commande 41 ainsi que le second récepteur 44 pour recevoir les signaux 42 adaptés pour localiser la télécommande 12.

Selon un troisième mode de réalisation, qui diffère du premier mode de réalisation uniquement par ce qui suit, le dispositif de supervision 28 comprend en outre le second récepteur 44 et les moyens d'autorisation 32, tandis que l'équipement 10 ne comprend ni le second récepteur 44, ni les moyens d'autorisation 32. Dans ce mode de réalisation, le dispositif de supervision 28 est adapté pour recevoir les signaux 41 représentant une commande et les signaux 42 de localisation.

Dans une variante du troisième mode de réalisation, le second récepteur 44 recevant les signaux 42 de localisation est une couronne composée des puces RFID, la couronne s'étendant à la périphérie de la salle d'opération 8, par exemple à proximité du plafond de la salle d'opération 8.

Le système de gestion 6 selon l'invention est adapté pour sécuriser la communication entre les télécommandes 12 et l'équipement 10 d'un bloc opératoire 4. Notamment, le système de gestion 6 évite qu'un premier utilisateur pilote fortuitement l'équipement 10 utilisé par un deuxième utilisateur dans une salle d'opération 8 autre que celle dans laquelle le premier utilisateur se trouve. Néanmoins, le système de gestion 6 selon l'invention ne limite pas le confort d'utilisation : il n'existe pas de lien physique entre l'utilisateur et les équipements 10, le système de gestion 6 évite des déplacements de l'équipe médicale et la communication entre les télécommandes 12 et les équipements 10 n'est pas gênée par des obstacles.

En variante non représentée, le système de gestion comprend le premier émetteur 24 qui est un émetteur dont la portée est modifiable et des moyens de réglage de la portée. Ces moyens de réglage de la portée règlent la portée de l'émetteur 24 de telle sorte qu'elle est plus courte lorsque la commande appartient à la première classe que lorsque la commande appartient à la seconde classe.

## Revendications

1. Système de gestion (6) d'équipement d'un bloc opératoire (4), le bloc opératoire (4) comprenant au moins deux salles d'opération (8) dont chaque salle d'opération (8) comprend au moins un équipement (10), le système de gestion (6) comprenant au moins une télécommande (12) adaptée pour piloter l'au moins un équipement (10) dans chacune des salles (8), le système de gestion (6) comprenant pour chaque télécommande (12) :
- des moyens d'association (14) associant la ou chaque télécommande (12) exclusivement à l'une des salles d'opération (8) de sorte à autoriser l'au moins une télécommande (12) à piloter l'au moins un équipement (10) dans la salle (8) associée et à inhiber que l'au moins une télécommande (12) pilote chaque équipement (10) dans chacune des autres salles d'opération (8), auxquelles l'au moins une télécommande (12) n'est pas associée et
- des moyens de modification (16) adaptés pour modifier les moyens d'association (14) de telle sorte que les moyens d'association (14) associent l'au moins une télécommande (12) exclusivement à une autre des au moins deux salles d'opération (8),
**caractérisé en ce que** le système de gestion (6) comprend une station d'association (18) et des moyens de liaison (22) de la télécommande (12) à la station d'association (18), les moyens de modification (16) étant adaptés pour modifier l'association lorsque la télécommande (12) est reliée à la station d'association (18) par les moyens de liaison (22), et **en ce que** la station d'association (18) comprend une interface homme - machine (43) adaptée pour laisser un utilisateur choisir l'une des salles d'opération (8) à laquelle il souhaite associer la télécommande (12).

2. Système de gestion (6) d'équipement d'un bloc opératoire (4) selon la revendication 1, comprenant en outre des moyens de communication (23) entre la ou chaque télécommande (12) et le ou chaque équipement (10) dans chaque salle d'opération (8) adaptés pour communiquer des signaux de commande (41) de la télécommande (12) aux ou à chaque équipement (10), et dans lequel les moyens de communication (23) définissent une première classe de commandes destinées à un équipement (10) constituée de commandes critiques et une seconde classe de commandes destinées à cet équipement (10) constituées d'autres commandes que les commandes critiques, et en ce qu'il est prévu des moyens d'inhibition (30) adaptés pour inhiber le pilotage de l'équipement (10) par la télécommande (12) lorsque l'équipement (10) appartient à une salle d'opération (8) autre que la salle (8) à laquelle la télécommande (12) est associée, mais uniquement lorsque la commande appartient à la première classe de commandes, mais non pas lorsque la commande appartient à la seconde classe de commandes.

3. Système de gestion (6) selon la revendication 2, dans lequel les moyens de communication (23) comprennent un premier émetteur (24) disposé dans chaque télécommande (12) et dans lequel chaque salle d'opération (8) comporte au moins un premier récepteur (26) correspondant, le premier émetteur (24) et le premier récepteur (26) utilisant une liaison de communication par radiofréquence afin de piloter l'équipement (10) de fa salle (8) associée.

4. Système de gestion (6) selon l'une quelconque des revendications 1 à 3, comprenant des moyens d'autorisation (32) qui sont adaptés pour autoriser la télécommande (12) à piloter l'équipement (10) dans la salle d'opération (8) à laquelle la télécommande (12) est associée uniquement lorsque la télécommande (12) se trouve à l'intérieur d'un périmètre défini, par exemple à l'intérieur de la salle (8) associée, et pour interdire à la télécommande (12) dé piloter l'équipement (10) dans la salle d'opération (8) à laquelle elle est associée lorsque la télécommande (12) se trouve en dehors du périmètre défini.

5. Système de gestion (6) selon la revendication 4 dans lequel les moyens d'autorisation (32) comprennent au moins l'un des suivants :
- un dispositif de localisation de la télécommande (12), le dispositif de localisation utilisant notamment un principe de triangulation ;
- un second émetteur (34) utilisant une liaison de communication par radiofréquence avec une portée plus courte que celle du premier émetteur (24) ;
- un second émetteur (34) utilisant une liaison de communication par ondes infrarouges vérifiant que la télécommande (12) pointe sur l'équipement (10) à commander ou sur le premier récepteur (26) correspondant ;
- un second émetteur (34) utilisant une liaison de communication par ondes ultrasoniques,
- dans le cas des revendications 2 et 4 prises ensembles, le premier émetteur (24) et des moyens de réglage de la portée, ce premier émetteur (24) étant un émetteur dont la portée est modifiable et les moyens de réglage de la portée réglant la portée de telle sorte qu'elle est plus courte lorsque la commande appartient à la première classe que lorsque la commande appartient à la seconde classe.

6. Système de gestion (6) selon l'une quelconque des revendications 1 à 5 comprenant une station de confirmation (20) adaptée pour confirmer l'association entre la télécommande (12) et la salle (8) et pour inhiber que la télécommande (12) commande l'équipement (10) de la salle (8) associée avant que l'association soit confirmée.

7. Système de gestion (6) selon au moins l'une des revendications 5 ou 6 dans lequel, selon le cas, la station d'association (18) et/ou la station de confirmation (20) sont adaptées pour communiquer avec la ou chaque télécommande (12) par une liaison du type « Bluetooth », en particulier « Bluetooth 2.0®» ou ultérieur, ou du type « ZigBee ».

8. Système de gestion (6) selon la revendication 3 comprenant un dispositif de supervision (28) disposé dans chaque salle d'opération (8), chaque dispositif de supervision (28) étant relié à au moins l'un équipement (10), et de préférence à au moins deux équipements (10), dans la salle d'opération (8), le dispositif de supervision (28) comprenant le premier récepteur (26) correspondant et le cas échéant des moyens d'association (14) dans lesquels est stockée une information d'association identifiant la ou les télécommande(s) (12) associée(s) à cette salle d'opération (8).

9. Utilisation d'un système de gestion (6) selon l'une quelconque des revendications précédentes pour gérer l'équipement (10) d'un bloc opératoire (4), le bloc opératoire (4) comprenant au moins deux salles d'opération (8), chaque salle d'opération (8) comprenant au moins un équipement (10), l'utilisation comprenant les étapes suivantes :
- au moyen des moyens d'association (14) associer la ou chaque télécommande (12) exclusivement à l'une des au moins deux salles d'opération (8) ;
- au moyen de la télécommande (12) piloter l'équipement (10) dans la salle d'opération (8) associée et éventuellement inhiber le pilotage par cette télécommande (12) de l'équipement (10) dans chacune des au moins deux salles (8) auxquelles la télécommande (12) n'est pas associée et
- modifier l'association de la ou de chaque télécommande (12) à l'une salle d'opération (8) des au moins deux salles (8) au moyen des moyens de modification (16) en associant la télécommande (12) à une autre des au moins deux salles d'opération (8).

## Patentansprüche

1. System zur Verwaltung (6) einer Anlage eines Operationstrakts (4), wobei der Operationstrakt (4) mindestens zwei Operationssäle (8) umfasst, von denen jeder Operationssaal (8) mindestens eine Anlage (10) umfasst, wobei das System zur Verwaltung (6) mindestens eine Fernbedienung (12) umfasst, die angepasst ist, um die mindestens eine Anlage (10) in jedem der Säle (8) zu steuern, wobei das System zur Verwaltung (6) für jede Fernbedienung (12) Folgendes umfasst:
- Verknüpfungsmittel (14), die die oder jede Fernbedienung (12) ausschließlich mit einem der Operationssäle (8) derart verknüpfen, dass die mindestens eine Fernbedienung (12) zum Steuern der mindestens einen Anlage (10) in dem verknüpften Saal (8) berechtigt wird und dass verhindert wird, dass die mindestens eine Fernbedienung (12) jede Anlage (10) in jedem der anderen Operationssäle (8) steuert, mit dem die mindestens eine Fernbedienung (12) nicht verknüpft ist, und
- Änderungsmittel (16), die angepasst sind, um die Verknüpfungsmittel (14) derart zu ändern, dass die Verknüpfungsmittel (14) die mindestens eine Fernbedienung (12) ausschließlich mit einem anderen der mindestens zwei Operationssäle (8) verknüpfen,
**dadurch gekennzeichnet, dass** das System zur Verwaltung (6) eine Verknüpfungsstation (18) und Mittel (22) zur Verbindung der Fernbedienung (12) mit der Verknüpfungsstation (18) umfasst, wobei die Änderungsmittel (16) angepasst sind, um die Verknüpfung zu ändern, wenn die Fernbedienung (12) mit der Verknüpfungsstation (18) durch die Verbindungsmittel (22) verbunden wird, und dadurch, dass die Verknüpfungsstation (18) eine Mensch-Maschine-Schnittstelle (43) umfasst, die angepasst ist, um einen Benutzer einen der Operationssäle (8) auswählen zu lassen, mit dem er die Fernbedienung (12) verknüpfen möchte.

2. System zur Verwaltung (6) einer Anlage eines Operationstrakts (4) nach Anspruch 1, das ferner Mittel (23) zur Kommunikation zwischen der oder jeder Fernbedienung (12) und der oder jeder Anlage (10) in jedem Operationssaal (8) umfasst, die angepasst sind, um Steuersignale (41) der Fernbedienung (12) an die oder jede Anlage (10) zu kommunizieren, und wobei die Kommunikationsmittel (23) eine erste Klasse von Befehlen, die für eine Anlage (10) bestimmt sind, die aus kritischen Befehlen besteht, und eine zweite Klasse von Befehlen definieren, die für diese Anlage (10) bestimmt sind, die aus anderen Befehlen als den kritischen Befehlen bestehen, und dadurch, dass Verhinderungsmittel (30) vorgesehen sind, die angepasst sind, um die Steuerung der Anlage (10) durch die Fernbedienung (12) zu verhindern, wenn die Anlage (10) zu einem anderen Operationssaal (8) als dem Saal (8) gehört, mit dem die Fernsteuerung (12) verknüpft ist, aber lediglich, wenn der Befehl zur ersten Klasse von Befehlen gehört aber nicht, wenn der Befehl zur zweiten Klasse von Befehlen gehört.

3. System zur Verwaltung (6) nach Anspruch 2, wobei die Kommunikationsmittel (23) einen ersten Sender (24) umfassen, der in jeder Fernbedienung (12) angeordnet ist, und wobei jeder Operationssaal (8) mindestens einen ersten entsprechenden Empfänger (26) umfasst, wobei der erste Sender (24) und der erste Empfänger (26) eine Hochfrequenz-Funkverbindung verwenden, um die Anlage (10) ihres verknüpften Saales (8) zu steuern.

4. System zur Verwaltung (6) nach einem der Ansprüche 1 bis 3, das Berechtigungsmittel (32) umfasst, die angepasst sind, um die Fernsteuerung (12) zum Steuern der Anlage (10) in dem Operationssaal (8), mit dem die Fernbedienung (12) verknüpft ist, lediglich dann zu berechtigen, wenn die Fernbedienung (12) sich innerhalb eines definierten Gebietes, zum Beispiel innerhalb des verknüpften Saales (8) befindet, und um das Steuern der Anlage (10) durch die Fernbedienung (12) in dem Operationssaal (8), mit dem sie verknüpft ist, zu verhindern, wenn die Fernsteuerung (12) sich außerhalb des definierten Gebietes befindet.

5. System zur Verwaltung (6) nach Anspruch 4, wobei die Berechtigungsmittel (32) mindestens eines von Folgendem umfassen:
- eine Vorrichtung zur Ortsbestimmung der Fernbedienung (12), wobei die Vorrichtung zur Ortsbestimmung insbesondere ein Triangulationsprinzip verwendet;
- einen zweiten Sender (34), der eine Hochfrequenz-Kommunikationsverbindung mit einer Reichweite verwendet, die kürzer ist als diejenige des ersten Senders (24);
- einen zweiten Sender (34), der eine Infrarotwellen-Kommunikationsverbindung verwendet, die überprüft, dass die Fernbedienung (12) auf die zu steuernde Anlage (10) oder auf den entsprechenden ersten Empfänger (26) gerichtet ist;
- einen zweiten Sender (34), der eine Ultraschallwellen-Kommunikationsverbindung verwendet,
- im Fall der Ansprüche 2 und 4 zusammen genommen, den ersten Sender (24) und Mittel zur Regelung der Reichweite, wobei dieser erste Sender (24) ein Sender ist, dessen Reichweite veränderlich ist, und die Mittel zur Regelung der Reichweite die Reichweite derart regeln, dass sie kürzer ist, wenn der Befehl zur ersten Klasse gehört als wenn der Befehl zur zweiten Klasse gehört.

6. System zur Verwaltung (6) nach einem der Ansprüche 1 bis 5, das eine Bestätigungsstation (20) umfasst, die angepasst ist, um die Verknüpfung zwischen der Fernbedienung (12) und dem Saal (8) zu bestätigen und um zu verhindern, dass die Fernbedienung (12) die Anlage (10) des verknüpften Saales (8) steuert, bevor die Verknüpfung bestätigt ist.

7. System zur Verwaltung (6) nach mindestens einem der Ansprüche 5 oder 6, wobei, je nach Fall, die Verknüpfungsstation (18) und/oder die Bestätigungsstation (20) angepasst sind, um mit der oder jeder Fernbedienung (12) durch eine Verbindung des Typs "Bluetooth", insbesondere "Bluetooth 2.0®" oder höher, oder des Typs "ZigBee" zu kommunizieren.

8. System zur Verwaltung (6) nach Anspruch 3, das eine Überwachungsvorrichtung (28) umfasst, die in jedem Operationssaal (8) angeordnet ist, wobei jede Überwachungsvorrichtung (28) mit mindestens einer Anlage (10) und vorzugsweise mit mindestens zwei Anlagen (10) in dem Operationssaal (8) verbunden ist, wobei die Überwachungsvorrichtung (28) den ersten entsprechenden Empfänger (26) und gegebenenfalls Verknüpfungsmittel (14) umfasst, in denen eine Verknüpfungsinformation gespeichert ist, die die Fernbedienung oder Fernbedienungen (12) identifiziert, die mit diesem Operationssaal (8) verknüpft ist oder sind.

9. Verwendung eines Systems zur Verwaltung (6) nach einem der vorhergehenden Ansprüche zum Verwalten der Anlage (10) eines Operationstrakts (4) wobei der Operationstrakt (4) mindestens zwei Operationssäle (8) umfasst, wobei jeder Operationssaal (8) mindestens eine Anlage (10) umfasst, wobei die Verwendung die folgenden Schritte umfasst:
- Verknüpfen der oder jeder Fernbedienung (12) ausschließlich mit einem der mindestens zwei Operationssäle (8) mittels der Verknüpfungsmittel (14);
- Steuern der Anlage (10) in dem verknüpften Operationssaal (8) mittels der Fernsteuerung (12) und gegebenenfalls Verhindern der Steuerung der Anlage (10) durch diese Fernbedienung (12) in jedem der mindestens zwei Säle (8), mit denen die Fernbedienung (12) nicht verknüpft ist, und
- Ändern der Verknüpfung der oder jeder Fernbedienung (12) mit dem einen Operationssaal (8) von den mindestens zwei Sälen (8) mittels der Änderungsmittel (16) durch Verknüpfen der Fernbedienung (12) mit einem anderen von den mindestens zwei Operationssälen (8).

## Claims

1. A management system (6) for managing equipment in an operating theater (4), the operating theater (4) having at least two operating rooms (8), with each operating room (8) having at least one piece of equipment (10), the management system (6) having at least one remote control (12) adapted to control at least one piece of equipment (10) in each of the rooms (8), the management system (6) comprising for each remote control (12):
· association means (14) associating the or each remote control (12) exclusively with one of the operating rooms (8) so as to authorize at least one remote control (12) to control at least one piece of equipment (10) in the associated room (8) and so as to inhibit the at least one remote control (12) from controlling each piece of equipment (10) in each of the other operating rooms (8) with which the at least one remote control (12) is not associated; and
· modification means (16) adapted to modify the association means (14) in such a manner that the association means (14) associate the at least one remote control (12) exclusively with another one of the at least two operating rooms (8),
**characterized in that** the management system (6) comprises an association station (18) and connection means (22) for connecting the remote control (12) to the association station (18), the modification means (16) being adapted to modify the association when the remote control (12) is connected to the association station (18) by the connection means (22), and **in that** the association station (18) comprises a man-machine interface (43) adapted to allow a user to select one of the operating rooms (8) with which the remote control (12) is to be associated.

2. A management system (6) for managing equipment in an operating theater (4) according to claim 1, further comprising communications means (23) for communication between the or each remote control (12) and the or each piece of equipment (10) in each operating room (8), the communications means being suitable for communicating command signals (41) from the remote control (12) to the or each piece of equipment (10), wherein the communications means (23) define a first class of commands for a piece of equipment (10) consisting of critical commands and a second class of commands for said piece of equipment (10) consisting of other commands than critical commands, and in that there is provided inhibition means (30) adapted to inhibit the control of the equipment (10) by the remote control (12) when the equipment (10) belongs to an operating room (8) other than the room (8) with which the remote control (12) is associated, but only when the command belongs to the first command class, but not when the command belongs to the second command class.

3. A management system (6) according to claim 2, wherein the communications means (23) comprise a first transmitter (24) arranged in each remote control (12), and wherein each operating room (8) comprises at least one corresponding first receiver (26), the first transmitter (24) and the first receiver (26) using a radiofrequency communications link for controlling the equipment (10) of the associated room (8).

4. A management system (6) according to any one of claims 1 to 3, comprising authorization means (32) that are adapted to authorize the remote control (12) to control the equipment (10) in the operating room (8) with which the remote control (12) is associated only when the remote control (12) is located within a defined perimeter, e.g. within the associated room (8), and to prevent the remote control (12) from controlling the equipment (10) in the operating room (8) with which it is associated when the remote control (12) is located outside the defined perimeter.

5. A management system (6) according to claim 4, wherein the authorization means (32) comprise at least one of the following:
· a locating device for locating the remote control (12), the locating device making use in particular of a triangulation principle;
· a second transmitter (34) using a radiofrequency communications link having range that is shorter than the range of the first transmitter (24);
· a second transmitter (34) using an infrared wave communications link requiring the remote control (12) to be pointed at the equipment (10) that is to be controlled or at the corresponding first receiver (26);
· a second transmitter (34) using an ultrasound wave communications link; and
· in the event that claims 2 and 4 are taken together, the first transmitter (24) and range adjustment means, the first transmitter (24) being a transmitter of adjustable range and the range adjustment means adjusting the range in such a manner that it is shorter when the command belongs to the first class than when the command belongs to the second class.

6. A management system (6) according to any one of claims 1 to 5, comprising a confirmation station (20) adapted to confirm the association between the remote control (12) and the room (8) and to inhibit the remote control (12) from controlling the equipment (10) of the associated room (8) before the association has been confirmed.

7. A management system (6) according to at least one of claims 5 or 6, wherein, depending on circumstances, the association station (18) and/or the confirmation station (20) is/are adapted to communicate with the or each remote control (12) via a link of the "Biuetooth□" type, in particular of the "Bluetooth 2.0□" or later type, or of the "ZigBee" type.

8. A management system (6) according to claim 3, comprising a supervisor device (28) arranged in each operating room (8), each supervisor device (28) being connected to at least one piece of equipment (10) and preferably to at least two pieces of equipment (10) in the operating room (8), the supervisor device (28) comprising the corresponding first receiver (26) and, where appropriate, association means (14) storing information associating the identifier of the or each remote control (12) associated with the operating room (8).

9. The use of a management system (6) according to any preceding claim for managing the equipment (10) of an operating theater (4), the operating theater (4) comprising at least two operating rooms (8), each operating room (8) comprising at least one piece of equipment (10), the use comprising the following steps:
· using the association means (14) to associate the or each remote control (12) exclusively with one of the at least two operating rooms (8);
· using the remote control means (12) to control the equipment (10) in the associated operating room (8) and optionally to inhibit that remote control (12) from controlling the equipment (10) in each of the at least two rooms (8) with which the remote control (12) is not associated; and
· modifying the association of the or each remote control (12) with one of the operating rooms (8) of the at least two rooms (8) by using the modification means (16) to associate the remote control (12) with another one of the at least two operating rooms (8).
